# EUROPEAN PATENT APPLICATION

(11) **EP 3 944 859 A1**
(43) Date of publication of application: **02.02.2022**
(21) Application number: 20305873.0
(22) Date of filing: 30.07.2020
(51) Int. Cl.: A61K 31/519, A61K 39/395, A61P 9/00, A61P 35/00, A61K 45/06

(54) **METHOD FOR TREATING IMMUNE TOXICITIES INDUCED BY IMMUNE CHECKPOINT INHIBITORS**

(71) Applicant: Assistance Publique Hôpitaux de Paris, 75004 Paris (FR); Sorbonne Université, 75006 Paris (FR); INSERM (Institut National de la Santé et de la Recherche Médicale), 75013 Paris (FR)
(72) Inventor: ALLENBACH, Yves, 75018 PARIS (FR); SALEM, Joe-Elie, 75015 PARIS (FR); ANQUETIL, Céline, 75015 PARIS (FR)
(74) Representative: Flesselles, Bruno F.G.

(57) **Abstract**

The invention relates to the use of a JAK inhibitor for treating or preventing adverse events in patient treated with an immune checkpoint inhibitor, or for treating cancer in combination with an immune checkpoint inhibitor.

## Description

Checkpoint inhibitor therapy is a form of cancer immunotherapy that has revolutionized oncology treatment. It consists in targeting immune checkpoints, which are key regulators of the immune system that stimulate or inhibit its actions. Checkpoint therapy in oncology aims at blocking inhibitory checkpoints, which tumors use to protect themselves from attacks by the immune system, hence restoring immune system function and fighting the tumor.

The currently approved immune checkpoint inhibitors (ICI) target the molecules CTLA4 (Cytotoxic T-Lymphocyte associated protein 4), PD-1 (the transmembrane programmed cell death 1 protein, also called PDCD1 and CD279), and PD-L1, which is the PD-1 ligand (or CD274). PD-1 acts as a key regulatory role on T cell activities, and cancer-mediated upregulation of PD-L1 on the cancer cell surface may inhibit T cells recognition and action against these cells. Antibodies against PD-1 or PD-L1 block the interaction between these proteins and allow T-cells to attack the tumor.

Another inhibitory checkpoint targeted in oncology is CTLA-4 (CD152), a protein receptor constitutively expressed in regulatory T cells and upregulated in conventional T cells after activation. It acts as an inhibitory switch of the immune system when it binds to CD80 or CD86 expressed on the surface of antigen-presenting cells.

However, the activation of the immune system after use of such immune checkpoint inhibitors may also lead to immune related adverse events (irAE) affecting potentially any organ.

Although rare, fulminant, and fatal irAE on ICI may occur in ∼0.36-1.23% of treated patients (Wang et al. JAMA Oncol 2018;4:1721-8). Severe and fatal toxicities are more common with anti-CTLA4 therapies, particularly when combined with PD1 or PDL1 blockers. ICI induced myocarditis occurs rarely (<1%) but is the irAE with the highest fatality rate. In the largest case-series of 122 myocarditis cases worldwide, the fatality rate was reported to be 50% with an earlier onset and higher fatality rate with ICI combination therapy versus monotherapy (Salem et al. Lancet Oncol 2018;19:1579-89). Interestingly, ICI myocarditis occurred generally after few ICI doses (n=1-3) and were often associated with concurrent muscular, pulmonary and hepatic irAE, including myositis (25%) with a peculiar phenotype often associated with oculomotor and diaphragmatic dysfunction. A main contributing cause of death in ICI myocarditis is early progressive and refractory cardiac electrical instability (heart blocks and ventricular arrhythmias) and cardiac dysfunction leading to cardiogenic shock, often resistant to intense immunosuppression.

While rigorous studies for the treatment of irAEs have not been performed, consensus guidelines recommend initial treatment with high-dose corticosteroids with progressive tapering and holding ICI (Brahmer et al, J Clin Oncol 2018;36:1714-68). Corticosteroids doses range from bolus of 0.5-2mg/kg/day of prednisone up to 1g/day methylprednisolone, depending on severity of clinical presentation. If symptoms and laboratory findings do not improve or worsen with steroids, other immunosuppressive drugs (mycophenolate-mofetil, cyclophosphamide, cyclosporine, tacrolimus, mTors inhibitors, methotrexate, azathioprine, antithymocyte globulin, alemtuzumab, infliximab, and rituximab) can be considered, depending on organs affected. In case of associated myositis and/or myasthenia gravis, intravenous immunoglobulin or plasmapheresis can be considered when presentations are severe and/or corticosteroid-resistant. In a subset of patients with fulminant, and chronic toxicities, however, available immunosuppressants produce suboptimal results (i.e. the 1.23% of patients who die from PD1/CTLA4 blockade induced toxicities).

The invention thus relates to a JAK inhibitor for use thereof for the treatment of immune-related adverse events induced by a treatment with an immune checkpoint inhibitor.

In particular, said immune-related adverse events induced by a treatment with an immune checkpoint inhibitor is not toxidermia (cutaneous lesions, such as skin rashes and/or ulcerations), colitis or arthritis.

The JAK inhibitor can also be used to prevent occurrence or recurrence of an immune-related adverse event induced by a treatment with an immune checkpoint inhibitor. In this embodiment, the JAK inhibitor is used in combination with the immune checkpoint inhibitor. In this embodiment, the patient receiving the ICI doesn't have any adverse event when the JAK inhibitor is administered. In other words, the administration of the JAK inhibitor is performed in the absence of any adverse event. The purpose is to avoid occurrence of such adverse events.

Such use is particularly adapted when the adverse event is a *de novo* event, *i.e.* is not related to a preexisting immune condition. In this embodiment, it is preferred when the patient has not been diagnosed with an auto-immune disease prior to the inset of the treatment with the immune checkpoint inhibitor.

The proposed use is also of particular interest when the adverse event is T-cell and/or macrophage driven, i.e. involving infiltration of CD4+ and/or CD8+ T cells and/or CD68+ macrophages, with minimal or no implication of antibodies (no presence of antibody deposits).

By JAK inhibitor, it is intended to designate a molecule that inhibits the activity of one or more of the Janus kinase family of enzymes, thereby interfering with the JAK-STAT signaling pathway. Some examples of effect of the JAK/STAT signaling pathway are production of colony-stimulating factor, prolactin, growth hormone, and many cytokines. Janus kinases (or JAKs) is an intracellular, non-receptor tyrosine kinase family consisting of four different subtypes, namely JAK1, JAK2, JAK3, and TYK2. JAK1, JAK2, and TYK2 are ubiquitously expressed, while JAK3 is mainly localized in hematopoietic cells.

JAK inhibitors have been proposed to be used for treating various diseases such as autoimmune diseases (in particular psoriasis, rheumatoid arthritis, inflammatory bowel disease, Sjogren's syndrome, Behçet's disease, multiple sclerosis, or systemic lupus erythematosus), immunological diseases or inflammatory diseases, central nervous system disorders, organ transplants, hyperproliferative diseases including cancer and myeloproliferative diseases, viral diseases, metabolic diseases and vascular diseases, pulmonary arterial hypertension, asthma, or chronic obstructive pulmonary disease. Tofacitinib has also been used in therapy for ulcerative colitis. All these use are linked to diseases that are not drug induced, and could be qualified as spontaneous.

Many inhibitors have been developed over the past 20 years, some of which being ubiquitous (inhibiting the activity of more than one kinases of the JAK family), and other being more specific (targeting essentially the kinase activity of only one member of the JAK family).

In an embodiment, the JAK inhibitor is a TYK2 inhibitor.

Inhibitors of TYK2 are described in the art, in particular in various patents such as the ones mentioned in Norman (Expert Opin. Ther. Patents (2014) 24(3):361-368), Norman (Expert Opin. Ther. Patents (2012) 22(10):1233-1249), Menet (Pharm. Pat. Anal. (2014) 3(4), 449-466) or He et al. (Expert Opin Ther Pat. 2019 Feb;29(2): 137-149).

In further details, DE102009015070A1 discloses N-[3-(4-aminopyrimidin-2-yl)aminophenyl] urea derivatives. WO2011113802 describes derivatives of 3H-imidazo[4,5-c]pyridin-4-amines and 7H-purin-6-amines, WO2012035039 describes derivatives of thiazolo[5,4-c]pyridin-4-amines and thiazolo[4,5-d]pyrimidin-7-amines and WO2012066061 describes derivatives of 2H-pyrazolo[4,3-c]pyri-din-2-amines and 2H-pyrazolo[3,4-d]pyrimidin-4-amines. WO2012000970 describes derivatives of aryl-substituted bicyclic amines, 5-phenyl-[1,2,4]triazolo[1,5-a]pyri-din-2-amine (triazolopyridine Tyk2 inhibitors), WO2012062704 describes derivatives of 2-aminopyrimi-dine, 2-amino-1,3,5-triazine and 2-aminopyridine (Monocyclic Tyk2 inhibitors), WO2013174895 describes pyrimidine based analogs as Tyk2 inhibitors, WO2015032423 describes substituted 5-amino-2-phenyloxazole-4-carboxamide which show a high selectivity fpr Tyk2 over Jak1, Jak2, and Jak3.

WO2015016206, WO2013146963 and WO2013125543 describe 2,4-diaminopyridine compounds (WO2013125543 relates to derivatives based on 1,5-dihydro-4H-pyrazolo[4,3-c]pyridine-4-one scaffold and WO2013146963 pertains to derivatives of 1-(2-arylaminopyrimidin-4-yl)-pyrrolidin-2-one), JP 2016-65023 describes 3-amino-1,5-dihydro-4H-pyrazolo[4,3-c]pyridin-4-one analogs. US20150299139, WO2015069310. US9505748, and WO20180162889 disclose compounds based on an imidazo[1,2-b]pyridazine scaffold. One can also mention WO2015089143, WO2017087590, WO2018067432 and WO2018093968. In particular, the compounds of WO2015016206 show a high Tyk2 inhibitory effect.

EP2634185 pertains to derivatives of 5-anilino-2-(2-halophenyl)-oxazole-4-carboxamide with nanomolar activity against Tyk2 and high selectivity over JAK1, JAK2 and JAK3.

In an embodiment, the JAK inhibitor is a JAK3 inhibitor.

JAK3 inhibitors are described in the art, in particular in patents and applications mentioned in Dymock and See (Expert Opin Ther Pat. 2013 Apr;23(4):449-501) or Wilson (Expert Opin. Ther. Patents (2010) 20(5):609-623).

Of particular interest is Tofacitinib (3-[(3*R*,4*R*)-4-Methyl-3-[methyl(7*H-*pyrrolo[2,3-d]pyrimidin-4-yl)amino]piperidin-1-yl]-3-oxopropanenitrile) that is currently used to treat rheumatoid arthritis, psoriatic arthritis, ankylosing spondylitis and ulcerative colitis. It is disclosed in WO200142246 or US6956041.

One can also cite Decernotinib ((2*R*)-2-Methyl-2-[[2-(1*H*-pyrrolo[2,3-b]pyridin-3-yl)pyrimidin-4-yl]amino]-*N*-(2,2,2-trifluoroethyl)butanamide) and PF-06651600 which are in clinical trials.

One can also cite Peficitinib (4-[[(1R,3S)-5-hydroxy-2-adamantyl]amino]-1H-pyrrolo[2,3-b]pyridine-5-carboxamide).

In an embodiment, the JAK inhibitor is a JAK2 inhibitor.

Inhibitors of JAK2 are known in the art, in particular in patents and applications mentioned in Dymock and See (Expert Opin Ther Pat. 2013 Apr;23(4):449-501) or Kiss et al (Expert Opin Ther Pat. 2010 Apr;20(4):471-95).

One can cite in particular Fedratinib (*N*-*tert*-Butyl-3-{5-methyl-2-[4-(2-pyrrolidin-1-yl-ethoxy)-phenylamino]-pyrimidin-4-ylamino}-benzenesulfonamide), disclosed in WO2007053452.

One can also cite Gandotinib (3-(4-Chloro-2-fluorobenzyl)-2-methyl-*N*-(5-methyl-1*H*-pyrazol-3-yl)-8-(morpholinomethyl)imidazo[1,2-*b*]pyridazin-6-amine).

Of interest is also Lestaurtinib ((5*S*,6*S*,8*R*)-6-Hydroxy-6-(hydroxymethyl)-5-methyl-7,8,14,15-tetrahydro-5*H*-16-oxa-4b,8a,14-triaza-5,8-methanodibenzo[b,h] cycloocta[jkl]cyclopenta[e]-as-indacen-13(6*H*)-one), or Pacritinib ((16*E*)-11-[2-(1-Pyrrolidinyl)ethoxy]-14,19-dioxa-5,7,26-triazatetracyclo[19.3.1.1^{2,6}.1^{8,12}]heptacosa-1(25),2(26),3,5,8,10,12(27),16,21,23-decaene).

In an embodiment, the JAK inhibitor is a JAK1 inhibitor.

Inhibitors of JAK1 inhibitors are described in particular in patent or applications mentioned in Norman (Expert Opin. Ther. Patents (2012) 22(10):1233-1249).

Oclacitinib (*N*-Methyl{trans-4-[methyl(7*H*-pyrrolo[2,3-*d*]pyrimidin-4-yl)amino] cyclohexyl}methanesulfonamide) is of particular interest.

Upadacitinib ((3*S*,4*R*)-3-Ethyl-4-(3*H*-imidazo[1,2-*a*]pyrrolo[2,3-*e*]pyrazin-8-yl)-*N*-(2,2,2-trifluoroethyl)pyrrolidine-1-carboxamide) is also of interest. It is described in WO2009152133.

Filgotinib (*N*-[5-[4-[(1,1-Dioxo-1,4-thiazinan-4-yl)methyl]phenyl]-[1,2,4]triazolo [1,5-a]pyridin-2-yl]cyclopropanecarboxamide) is also of interest.

One can also cite Abrocitinib (N-(cis-3-(Methyl(7H-pyrrolo(2,3-d)pyrimidin-4-yl)amino)cyclobutyl)propane-1-sulfonamide).

In an embodiment, the JAK inhibitor is a JAK1/JAK2 inhibitor.

Ruxolitinib ((3*R*)-3-Cyclopentyl-3-[4-(7*H*-pyrrolo[2,3-d]pyrimidin-4-yl)pyrazol-1-yl]propanenitrile) is a janus kinase inhibitor (JAK inhibitor) with selectivity for subtypes JAK1 and JAK2. It was described in WO2007070514.

One can also cite Baricitinib (2-[1-Ethylsulfonyl-3-[4-(7*H*-pyrrolo[2,3-d]pyrimidin-4-yl)pyrazol-1-yl]azetidin-3-yl]acetonitrile)

Momelotinib (*N*-(cyanomethyl)-4-{2-[4-(morpholin-4-yl)anilino]pyrimidin-4-yl}benzamide) is also of interest.

Other inhibitors of enzymes of the JAK family are also described in WO2011113802, WO2012035039, WO2012066061, WO2013041539, WO2011130146, WO2013055645, WO2012160464, WO2014000032, WO2010142752 and WO2012160464 and in Kettle et al (Expert Opinion on Therapeutic Patents, 27:2, 127-143 ; Expert Opinion on Therapeutic Patents, 27:2, 145-161).

One can also cite Cerdulatinib (4-(Cyclopropylamino)-2-[4-(4-ethylsulfonylpiperazin-1-yl)anilino]pyrimidine-5-carboxamide) which is an inhibitor of TYK2, JAK1, JAK2, JAK3, FMS, and SYK.

The JAK inhibitor can therefore be used in a method for treating, or in a method for preventing, occurrence of an adverse event induced by a treatment with an immune checkpoint inhibitor, comprising the step of administering an effective amount of the JAK inhibitor to a patient in need thereof. An effective amount, or therapeutic amount, is the amount sufficient to obtain beneficial or desired results, such as clinical results (remission of the symptoms of the immune related adverse events). The "effective amount" may depend upon the type of immune related adverse event, and upon the context in which it is being applied. In the context of the invention, an effective amount of a JAK inhibitor is, for example, an amount sufficient to achieve a reduction in the severity of the immune related adverse event, as compared to the response obtained without administration of the agonist.

As the immune related adverse event induced by the treatment with checkpoint inhibitors, one can cite, pneumonitis, hepatitis, hypophysitis, neurologic adverse effects (including encephalitis, myasthenia gravis, Guillain-Barre syndrome), adrenal adverse effect, myositis, myocarditis, hematologic adverse effects (including hemolytic anemia, immune thrombocytopenic purpura, and aplastic anemia), nephritis, pancreatitis, and type 1 diabetes. All these diseases can be linked to the administration of ICI.

In particular, the adverse effect is a *de novo* effect, meaning that it was not present in the patient, or had not been diagnosed, prior to onset of the treatment of with the ICI.

Use of the JAK inhibitor is of particular interest for the treatment of a myotoxicity (in particular an immune-mediated myotoxicity), in particular fulminant myocarditis or fulminant diaphragmatic myositis. Fulminant myocarditis (FM) is a peculiar clinical condition (more frequently associated with anti-PD1/PL1 antibodies) and is an acute form of myocarditis, whose main characteristic is a rapidly progressive clinical course with the need for hemodynamic support, which has been shown to be linked to the presence of selective clonal T-cell populations, identical to those present in tumors and skeletal muscle, infiltrated in the myocardium, this event thus being a T-cell-driven drug reaction. This T-cell-driven mechanism is characteristic for immune-mediated myotoxicity. In contrast, in immune-mediated colitis, the patho-mechanisms are different since innate immune responses are also involved attested by the key role of gut microbiota and neutrophylic polynuclear leucocytes.

This kind of fulminant myocarditis, when present in humans, doesn't mainly involve antibodies.

It is preferred when the JAK inhibitor is selected from the group consisting of ruxolitinib, tofacitinib, oclacitinib, baricitinib, peficitinib, fedratinib, upadacitinib, filgotinib, cerdulatinib, gandotinib, lestaurtinib, momelotinib, pacritinib and abrocitinib.

Ruxolitinib is of particular interest.

Tofacitinib is also of particular interest.

Baricitinib is also of particular interest.

The immune checkpoint inhibitor used for the patient's treatment before occurrence of the adverse effect is any such drug in this class.

In particular, one can cite:
PD-1 inhibitors : IgG4 PD1 antibody nivolumab, pembrolizumab, partalizumab (PDR001) developed by Novartis, pidilizumab developed by Cure Tech, AMP-224 or AMP-514 both developed by GlaxoSmithKline, cemiplimab developed by Regeneron and Sanofi, toripalimab developed by Shanghai Junshi, spartalizumab, developed by Novartis, cetrelimab (JNJ-63723283) developed by Janssen, or sasanlimab (PF-06801591) developed by Pfizer.
PD-L1 inhibitors: atezolizumab developed by Roche Genentech, avelumab developed by Merck Serono and Pfizer or durvalumab developed by AstraZeneca.
Anti-CTLA4: ipilimumab or tremelimumab.

The JAK inhibitor is used at a dosage similar to or up to 4 times higher than those dosages preconized by the manufacturer and according to good practice in the art. Choice of the appropriate dosage may be adapted by the physician depending on the severity of the clinical presentation and evolution on treatment.

As a matter of illustration, the current dosing of ruxolitinib is comprised between 5 mg and 20 mg twice a day, depending on the disease, and of the platelets amount. Tofacitinib is also administered at these kind of amounts (from 5 mg to 20 mg twice a day).

It is thus preferred when the JAK inhibitor is administered *via* multiple administrations, in particular multiple daily administrations. As a matter of illustration, the JAK inhibitor can be administered twice daily to the patient.

As indicated above, the dose administered to the patient is chosen so as to be therapeutically effective. It is thus possible to use higher amount of such products for the first administration in order to try to quickly diminish the adverse effects, and then lower the dose when the clinical status of the patient improves.

It is generally envisaged to limit administration of the JAK inhibitor to the patient, and to stop the treatment when the patient general condition has improved. In a specific embodiment, the treatment should last for a few weeks or maximum months. For instance, a treatment may last from about three to twenty weeks, generally about four to ten weeks. In this case, there would be multiple administration of the JAK inhibitor provided to the patient.

The JAK inhibitor is generally in a form suitable for oral administration. However, it may be in a form suitable for injectable administration. Preferably, such administration is a intravenous injection, intra-muscular or subcutaneous injection.

In specific embodiment, the JAK inhibitor is in the form of a slow release composition, which would enable decreasing the number of administrations.

Even though the examples excluded the use of an immunosuppressant, in the context of the COVID-19 pandemy, it may be of interest to use an immunosuppressant together with the JAK inhibitor for treating the adverse effects.

In combination with administration of JAK inhibitors, as described above, glucocorticoids at high dose (up to 1 g/day methylprednisone equivalent for few days) and or other immunossupressants may be also be used. These latter immunosuppressants are preferentially antimetabolites (such as mycofenolate mofetil, azathioprine, methotrexate), anti-calcineurin (also designated as calcineurin inhibitors) (ciclosporin, tacrolimus), mTOR inhibitors (sirolimus, temsirolimus, everolimus), anti-thymoglubin, intravenous immunoglobulin, interleukin-6 inhibitors (tocilizumab, siltuximab, sarilumab, sirukumab, olokizumab, clazakizumab), interleukin-1 pathway inhibitors (anakinra, rilonacept, canakinumab), TNF-α inhibitors, CTLA4 agonist or anti CD28. Other compounds that can be used also include basiliximab (chimeric mouse-human monoclonal antibody) or daclizumab (both binding to the α chain (CD25) of the IL-2 receptor of T cells), tocilizumab, also known as atlizumab, humanized monoclonal antibody against the interleukin-6 receptor (IL-6R), and alemtuzumab (monoclonal antibody that binds to CD52).

It is of particular interest to use corticoids. In another embodiment, it is of interest to use a CTLA4 agonist, in particular abatacept or belatacept.

It is also possible to use plasmapheresis to clear immune checkpoint inhibitor drug levels in the circulation. In this case, the scheme of administration of the JAK inhibitor (in particular Ruxolitinib or Tofacitinib) needs to be adapted.

The invention also relates to the JAK inhibitor for use as indicated above, it is administered with another immunosuppressant, or any other drug as disclosed below, including glucocorticoids, antimetabolites (such as mycofenolate mofetil, azathiatrine, methotrexate), calcineurin inhibitors (ciclosporin, tacrolimus), mtor inhibitors (sirolimus, temsirolimus, everolimus), anti-thymoglubin, intravenous immunoglobulin, interleukin-6 inhibitors (tocilizumab, siltuximab), anti CD52 (alemtuzumab),, anti CD25 (basiliximab, daclizumab), interleukin-1 pathway inhibitors (anakinra, rilonacept, canakinumab), TNF-α inhibitors, and anti CD28. Said co-administration can be simultaneous, separate or sequential (spread out over time).

The invention also relates to a composition containing a JAK inhibitor and an immunosuppressant or any other drug as disclosed above, for simultaneous, separate or sequential (spread out over time) use thereof in the treatment or prevention of an adverse event induced by a treatment with an immune checkpoint inhibitor.

The invention also relates to a method for treating or preventing an adverse event induced by a treatment with an immune checkpoint inhibitor, comprising administering a therapeutically active amount of a JAK inhibitor to a subject in need thereof, with a simultaneous, separate or sequential (spread out over time) administration of an effective amount of an immunosuppressant to the subject.

The invention also relates to a JAK inhibitor for use thereof in the treatment of an adverse event induced by a treatment with an immune checkpoint inhibitor, in a patient in need thereof, wherein said patient has been subject to a plasmapheresis prior to administration of the JAK inhibitor. As indicated above, such plasmapheresis makes it possible to clear immune check-point inhibitors levels in the circulation of the patient.

The invention also relates to a method for treating a subject in need thereof, wherein said subject present an adverse effect induced by a treatment with an immune checkpoint inhibitor, comprising performing a plasmapheresis to the subject (so as to clear immune checkpoint inhibitors levels in the circulation of the subject) and administering an effective amount of a JAK inhibitor (alone or with another immunosuppressant) prior and after plasmapheresis.

The invention also relates to use of a JAK inhibitor as disclosed above, for the preparation of a medicament intended to treat or prevent an adverse event induced by a treatment with an immune checkpoint inhibitor in a patient. This medicament can also be a combination comprising an immunosuppressant as disclosed above.

The invention makes it possible to prevent the occurrence or recurrence of severe immune-related adverse events related to Immune Checkpoint Inhibitors (ICI-irAE). Indeed, the results of transcriptomics analysis reported herein show that ICIinduced myositis differs from spontaneous inflammatory myopathies in terms of gene expressions. It is thus envisaged to combine the administration of a JAK inhibitor and an ICI.

The invention thus relates to a composition containing a JAK inhibitor and an immune checkpoint inhibitor, for simultaneous, separate or sequential (spread out over time) use thereof in the treatment of cancer.

The invention also relates to a method for treating a cancer in a subject, comprising administering a therapeutically active amount of a JAK inhibitor to a subject in need thereof, with a simultaneous, separate or sequential (spread out over time) administration of an effective amount of an immune checkpoint inhibitor to the subject.

The cancer is, in particular, selected from the group consisting of bladder carcinoma, breast carcinoma, cervical carcinoma, cholangiocarcinoma, colorectal carcinoma, gastric sarcoma, glioma, lung carcinoma, lymphoma, acute and chronic lymphoid and myeloid leukemias, melanoma, multiple myeloma, osteosarcoma, ovarian carcinoma, pancreatic carcinoma, prostate carcinoma, stomach carcinoma, kidney carcinoma, a head and neck tumor, and a solid tumor.

In particular, it is possible to associate nivolumab and ruxolitinib, notably for treating pulmonary adenocarcinoma (even when metastatic).

### EXAMPLES

### Example 1

A 67-year-old man was treated for metastatic pulmonary adenocarcinoma with nivolumab as second-line therapy but developed myalgia, diplopia and bilateral ptosis after 2 treatments with nivolumab (4 weeks after treatment initiation).

Myositis was suspected due to increased creatine kinase levels and confirmed by muscle biopsy. Myocarditis was confirmed with troponin increase, premature ventricular contractions and deterioration of left-ventricular ejection fraction with no signs of acute coronary syndrome on angiogram.

The patient was treated with glucocorticoids and one exchange of plasmapheresis. However, due to positive SARS-Cov-2 infection, detected during hospitalization, and the uncertainty of glucocorticoids use for COVID-19 at that time, corticosteroids were tapered.

Ruxolitinib was initiated (15mg twice daily), with prompt decrease in troponin and improvement of oculomotor disorders. Ruxolitinib was continued for 1 month at 15mg twice daily, with complete resolution of myotoxicity; there was no evidence of tumor progression at 3 months.

Immune-checkpoint inhibitors (ICI)-associated myotoxicities (myocarditis and myositis) are infrequent but potentially fatal immune-related adverse events (irAE). Pathologically, ICI-myotoxicities are characterized by muscle infiltration of macrophages and T lymphocytes; the mechanism of muscle death is partly due proinflammatory cytokine production such as interferons.

Ruxolitinib, an oral selective inhibitor of JAK1 and JAK2 resulted in symptomatic relief of myotoxicity with no effect on tumor progression in follow-up.

This illustrates a role for JAK-inhibitors in the treatment of severe ICI-irAE.

### Example 2

RNA-sequencing of muscle tissue was performed to identify the pathological mechanisms involved in ICI-induced myositis compared to normal tissue or other idiopathic inflammatory myopathies (paraneoplastic dermatomyositis, immune-mediated necrotizing myopathy, inclusion body myositis). A total of 30 samples (5 groups of 6 patients) were studied using unsupervised analysis, differentially expressed gene analysis, and pathways analysis.

ICI-induced myositis clustered in an unique subgroup different from both controls and patients with other type of myositis. This results demonstrate that ICI-induced myositis pathomechanisms (genes expression) are specific.

ICI-induced myositis represented a specific cluster with more than 6 000 genes differentially expressed compared to healthy controls. A gene set enrichment analysis was performed on the up and down-regulated genes. In the top 10 identified pathways, the interferon gamma-mediated signaling pathways and the type I interferon signaling pathways were found, showing the importance of JAK-STAT pathway.

## Claims

1. JAK inhibitor for use thereof for the treatment of immune-related adverse events induced by a treatment with an immune checkpoint inhibitor, wherein the related adverse event is not toxidermia, colitis or arthritis.

2. JAK inhibitor for use thereof for the prevention of immune-related adverse events induced by a treatment with an immune checkpoint inhibitor.

3. JAK inhibitor for use according to claim 1 or 2, wherein the adverse event is an immune-mediated disease selected from the group consisting of myocarditis, , pneumonitis, hepatitis, hypophysitis, neurologic adverse effect, adrenal adverse effect, myositis, hematologic adverse effect, pancreatitis, endocrinological adverse effect, and nephritis.

4. JAK inhibitor for use according to any one of claims 1 to 3, wherein the adverse event is a *de novo* event, not related to a preexisting immune condition in the patient treated with the immune checkpoint inhibitor.

5. JAK inhibitor for use according to any one of claims 1 to 4, wherein the adverse event is an immune-mediated myotoxicity, in particular fulminant myocarditis or fulminant diaphragmatic myositis.

6. JAK inhibitor for use according to any one of claims 1 to 5, wherein the JAK inhibitor is selected from the group consisting of ruxolitinib, tofacitinib, oclacitinib, baricitinib, peficitinib, fedratinib, upadacitinib, filgotinib, cerdulatinib, gandotinib, lestaurtinib, momelotinib, pacritinib and abrocitinib.

7. JAK inhibitor for use according to any one of claims 1 to 6, wherein the checkpoint inhibitor is selected from the group consisting of PD-1 inhibitors, and/or PD-L1 inhibitors and/or anti-CTLA4.

8. JAK inhibitor for use according to claim 7, wherein the checkpoint inhibitor is a PD-1 inhibitor selected from the group consisting of nivolumab, pembrolizumab, partalizumab, pidilizumab, AMP-224, AMP-514, cemiplimab, toripalimab, spartalizumab, cetrelimab and sasanlimab.

9. JAK inhibitor for use according to claim 7, wherein the checkpoint inhibitor is a PD-L1 inhibitor selected from the group consisting of atezolizumab, avelumab and durvalumab.

10. JAK inhibitor for use according to claim 7, wherein the checkpoint inhibitor is a anti-CTLA4 selected from the group consisting of ipilimumab and tremelimumab

11. JAK inhibitor for use according to any one of claims 1 to 10, wherein multiple administrations of the JAK inhibitor are provided to the patient.

12. JAK inhibitor for use according to any one of claims 1 to 11, wherein said JAK inhibitor is administered during three to twenty weeks.

13. JAK inhibitor for use according to any one of claims 1 to 12, **characterized in that** it is in the form of a slow release composition.

14. Composition containing a JAK inhibitor and an immune checkpoint inhibitor, for simultaneous, separate or sequential (spread out over time) use thereof in the treatment of cancer.

15. Composition containing a JAK inhibitor and an immunosuppressant, for simultaneous, separate or sequential (spread out over time) use thereof in the treatment or prevention of an adverse event induced by a treatment with an immune checkpoint inhibitor.
